# EUROPÄISCHE PATENTSCHRIFT

(11) **EP 0 697 023 B1**
(45) Veröffentlichungstag und Bekanntmachung des Hinweises auf die Patenterteilung: **25.11.1998**
(21) Anmeldenummer: 94915554.3
(22) Anmeldetag: 28.04.1994
(51) Int. Cl.: C07H 13/04, C07K 9/00

(54) **3-(AMINOACYL-AMINO)-SACCHARIDE UND VERFAHREN ZU IHRER HERSTELLUNG**
3-(AMINOACYL-AMINO)-SACCHARIDES AND METHOD FOR PREPARING THEM
3-(AMINOACYL-AMINO)-SACCHARIDES ET LEUR PROCEDE DE PREPARATION

(30) Priorität: 03.05.1993 DE 4314407
(43) Veröffentlichungstag der Anmeldung: 21.02.1996
(73) Patentinhaber: Verein der Zuckerindustrie, D-53113 Bonn (DE)
(72) Erfinder: BUCHHOLZ, Klaus, D-38124 Braunschweig (DE); NOLL-BORCHERS, Martina, D-45476 Mülheim an der Ruhr (DE); PIETSCH, Martina, D-47259 Duisburg (DE)
(74) Vertreter: Grussdorf, Jürgen, Dr.
(86) Internationale Anmeldenummer: EP9401339
(87) Internationale Veröffentlichungsnummer: WO9425474

(56) Entgegenhaltungen:
- US-A- 4 216 208
- ANGEW. CHEM., Bd.99, 1987, WEINHEIM, DE Seiten 297 - 311 V. H. KUNZ 'Synthese von Glycopeptiden, Partialstrukturen biologischer Erkennungskomponenten.' in der Anmeldung erwähnt
- CARBOHYDRATE RESEARCH, Bd.112, 1983, AMSTERDAM, NL Seiten 233 - 239 V. L. L'VOV ET AL '3-[(N-acetyl-L-seryl)am ino]-3,6-dideoxy-D-glucose: A novel constituent of a bacterial antigenic polysaccharide.' in der Anmeldung erwähnt

## Beschreibung

Die Erfindung betrifft 3-(Aminoacyl-amino)-saccharide und Verfahren zu ihrer Herstellung aus 3-Amino-allo-saccharose.

Verknüpfungen von Aminosäuren und Sacchariden liegen hauptsächlich in Glycopeptiden bzw. Glycoproteinen und in geringem Anteil auch in bakteriellen Lipopolysacchariden vor. Bei Glycopeptiden handelt es sich um Partialstrukturen, die als Verknüpfungsregion von Sacchariden und Proteinen in Glycoproteinen vorkommen. Glycoproteine, die in löslicher Form im Blut und in zahlreichen Sekreten sowie in fixierter Form in Membrandoppelschichten zu finden sind, haben in letzter Zeit an Interesse gewonnen, seit ihre Funktion in biologischen Steuerungsprozessen erkannt und untersucht wurde. In vielen Fällen dienen die Kohlenhydrat-Seitenketten als Erkennungssignal.

Die Art der kovalenten Bindung zwischen Protein und Kohlenhydrat-Seitenkette unterscheidet sich trotz der großen Vielzahl der natürlich vorkommenden Glycoproteine jedoch kaum, was durch die Biosynthese der Glycoproteine bedingt ist. Die beiden Komponenten sind über eine glycosidische Bindung verknüpft, wobei man N-Glycoproteine und O-Glycoproteine unterscheidet. Bei den N-Glycoproteinen ist meist die Seitenketten-Amidgruppe eines Asparagin-Bausteins mit 2-Acetamido-2-desoxy-D-glucose β-N-glycosidisch verknüpft. Daneben sind in neuerer Zeit jedoch auch N-Glycosyl- und N-Galactosyl-Strukturen gefunden worden. Bei den O-Glycoproteinen liegt meist eine α-O-glycosidische Verknüpfung von 2-Acetamido-2-desoxy-D-galactose oder eine β-O-glycosidische Verknüpfung von D-Xylose mit einer Hydroxylgruppe von Serin oder Threonin vor (H. Paulsen, Synthesen, Konformationen und Röntgen-strukturanalysen von Saccharidketten der Core-Regionen von Glycoproteinen, Angew. Chem. 102 (1990) 851-867; H. Kunz, Synthese von Glycopeptiden - Partialstrukturen biologischer Erkennungskomponenten, Angew. Chem. 99 (1987) 297-311; J. Montreuil, Primary Structure of Glycoprotein Glycans, Adv. Carbohydr. Chem. Biochem. 37 (1980) 157-223).

Aus dem US-P 4,216,208 sind N-Acylderivate des 2-Glucosamins bekannt, welche als antineoplastische Mittel in der Antitumor-chemotherapie genutzt werden können. Als Acylgruppe wird dabei eine m-Di(2-chlorethyl)-amino-L-phenylalaningruppe, die gegebenenfalls noch durch weitere Aminosäuren peptidartig substituiert ist, eingesetzt. Unsubstituiertes L-Phenylalanin erweist sich als wirkungslos.

Auch in Lipopolysacchariden bakteriellen Ursprungs wurden vereinzelt Aminosäuren nachgewiesen, die über eine Amidbindung mit Aminozuckern verknüpft sind, z.B. N-Acetylglycin, das über die Aminogruppe des 4-Amino-4,6-didesoxy-D-glycopyranosyl-Rests an die O-spezifische Seitenkette des Lipopolysaccharids von Shigella dysenteriae Typ 7 gebunden ist (Y. A. Knirel et al., Carbohydr. Res. 179 (1988) 51-60), oder N-Acetyl-L-serin, das über die Aminogruppe des 3-Amino-3,6-didesoxy-D-glucopyranosyl-Rests mit der O-spezifischen Seitenkette der Lipopolysaccharids von Escherichia coli 0114 verknüpft ist (V. L. L'vov et al., Carbohydr. Res. 112 (1983) 233-239). Diese Stoffe wirken als Antigene.

Die Erfindung bezieht sich auf neue 3-(Aminoacyl-amino)-saccharide der folgenden Formel I, 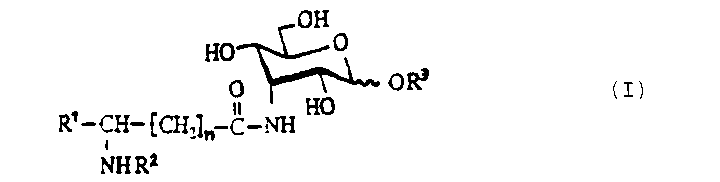
in der R¹ Wasserstoff, eine Carboxylgruppe oder eine Phenylgruppe oder eine Alkylgruppe mit 1 - 10 C-Atomen, die ggf. durch eine Phenyl-, Carboxyl-, Hydroxyl-, Mercapto- oder Aminogruppe substituiert ist, wobei die genannten Substituenten ggf. mit Schutzgruppen versehen sind, bedeutet,
R² Wasserstoff, eine in der Peptidchemie übliche Aminoschutzgruppe oder eine Peptidgruppe ist und
R³ Wasserstoff oder einen Fructosylrest darstellt,
n = 0 oder 1 ist,
insbesondere die 3-(L-Aminoacyl-amino)-D-allopyranosyl-β-D-fructofuranoside, im folgenden mit 3-(Aminoacyl-amino)-allosaccharose bezeichnet, und 3-(L-Aminoacyl-amino) -D-allopyranosen. Die Erfindung umfaßt ferner Herstellungsverfahren für diese Stoffe ausgehend von 3-Amino-3-desoxy-D-allo-pyranosyl-β-D-fructofuranosid, kurz 3-Amino-allo-saccharose genannt.

Bei den erfindungsgemäßen 3-(Aminoacyl-amino)-allo-sacchariden handelt es sich um Stoffe, in denen die Carboxylgruppe einer Aminosäure über eine Säureamidbindung an die Aminogruppe der 3-Amino-allo-saccharose gebunden ist. Diese Verbindungen enthalten somit im Gegensatz zu den Glycoproteinen keine glycosidische Bindung zwischen Kohlenhydrat und Aminosäure, sondern weisen eine ähnliche Verknüpfungsstruktur auf wie die oben erwähnten Lipopolysaccharide.

Die 3-(Aminoacyl-amino)-allo-saccharosen ermöglichen mit Hilfe der Methoden der Glycopeptidsynthese die Herstellung neuartiger Glycopeptide, in denen Kohlenhydrat und Protein nicht über eine N-glycosidische Bindung, sondern über eine Säureamidbindung verknüpft sind. Hierfür sind als Ausgangsverbindungen insbesondere saure und basische Aminosäuren geeignet, da der Aminosäurerest in den Aminosäure-sacchariden jeweils noch zwei funktionelle Gruppen enthält, also die Peptidkette an zwei Seiten aufgebaut werden kann.

Die Herstellung von Glycopeptiden ist normalerweise aufgrund der Polyfunktionalität beider Ausgangskomponenten sehr problematisch und erfordert meist den Einsatz von Schutzgruppen sowohl am Saccharid als auch an der Aminosäure bzw. dem Peptid. Durch den Einsatz der 3-Amino-allo-saccharose, die ohne Einführung von Schutzgruppen erhalten werden kann, ist es nun möglich, für die Synthese der 3-Aminoacylgruppe im Saccharidteil ganz auf Schutzgruppen zu verzichten.

Über die Abspaltung des Fructosylrests der 3-(Aminoacylamino)-allo-saccharosen kann man auch zu 3-(Aminoacyl-amino)allose gelangen, die ihrerseits zur Herstellung von Glycopeptiden dienen kann. In diesem Fall läßt sich sowohl eine Peptidkette aufbauen, indem an den Aminosäurerest weitere Aminosäuren geknüpft werden, als auch über die Allose eine Oligosaccharidkette herstellen.

Die erfindungsgemäßen Verbindungen sowie daraus hergestellte Derivate sind in der interdisziplinären Forschung und Anwendung einsetzbar, z. B. zur Aufklärung der biologischen Funktion von Glycoproteinen, zur Induktion von Antikörpern, zur Erforschung der biologischen Erkennung und Selektivität und zur Entwicklung von pharmazeutischen Wirkstoffen sowie als potentieller Hemmstoff für Enzyme und Mikroorganismen.

Die Herstellung der 3-(L-Aminoacyl)-amino-allo-saccharide geht von Saccharose aus. In dieses polyfunktionelle Molekül wird durch eine mikrobielle Oxidation mit Agrobacterium tumefaciens eine Ketogruppe am C-3 des Glucosylrests eingeführt, die anschließend mittels reduktiver Aminierung, z.B. mit Ammoniak, Hydroxylamin oder Hydrazin mittels Wasserstoff und Metallkatalysator, selektiv in eine Aminogruppe überführt werden kann. Nach chromatogaphischer Reinigung erhält man das Aminierungsprodukt 3-Amino-allo-saccharose (M. Pietsch, Dissertation TU-Braunschweig, 1993). Dieser Syntheseweg ist in ähnlicher Weise bereits für die Herstellung von Diaminen aus reduzierenden Sacchariden in EP 0 399 448 A2 beschrieben.

Die 3-Amino-allo-saccharose dient nun ihrerseits als Ausgangsprodukt für die 3-(Aminoacyl-amino)-allo-saccharide: Die Umsetzung gelingt überraschenderweise in Anlehnung an das von M. Kiyozumi et al., Carbohydr. Res. 14 (1970) 355-364 beschriebene Verfahren. Dazu wird die 3-Amino-allo-saccharose mit N-terminal geschützten Amino- und Diaminosäuren bzw. mit N- und C-terminal geschützten Aminodicarbonsäuren oder entsprechend geschützten Peptiden in einem Pyridin-Wasser-Gemisch unter Aktivierung der Carboxylfunktion der Aminosäure mittels N,N'-Dicyclohexylcarbodiimid (DCC) umgesetzt. Anschließend können die Schutzgruppen abgespalten werden. Eine Reinigung der Reaktionsprodukte ist z. B. durch Säulenchromatographie möglich. Der Strukturnachweis der gereinigten Substanzen erfolgt mittels ¹³C-NMR-Spektroskopie und Fast-Atom-Bombardment-Massenspektrometrie (FAB-MS).

Als Verfahren für die Herstellung der 3-(Aminoacylamino)saccharide werden folgende Schritte vorgeschlagen:
1. Oxidation von Saccharose z. B. mit Bakterien des Stammes Agrobacterium tumefaciens NCPPB 396 zu 3-Keto-saccharose.
2. Reduktive Aminierung der 3-Keto-saccharose sowie Aufarbeitung und Trennung des entstehenden Produktgemisches und Isolierung der 3-Amino-allo-saccharose.
3. N-Acylierung der 3-Amino-allo-saccharose mit Aminosäuren oder Peptiden mit einer aktivierten Carboxylgruppe, deren übrige Amino- und Carboxylgruppen mittels in der Peptidchemie üblichen Schutzgruppen ggf. geschützt sind.
4. Abspaltung einer oder mehrerer Schutzgruppen vom Aminosäurerest der Substanz und/oder des Fructosylrests aus der Saccharosegruppe sowie Isolierung und Reinigung des Produktes.

Für die Aktivierung der Carboxylgruppe wird Dicyclohexylcarbodiimid (DCC) bevorzugt, jedoch sind auch 1,2-Dihydro-2-ethoxychinolin-1-carbonsäureethylester (EEDQ) als Kopplungsreagenz und andere aktivierte Carboxylgruppen, wie Anhydride, Ester, Azide oder Halogenide, verwendbar, soweit sie nicht in größerem Maße mit den Hydroxylgruppen des Saccharids reagieren.

Als Aminoschutzgruppen sind alle aus der Peptidchemie bekannten Gruppen verwendbar, die sich in schonender Weise wieder abspalten lassen. Beispielsweise seien Benzyloxycarbonyl (CBZ), t-Butyloxycarbonyl (BOC) und Triphenylmethyl (Trt) genannt, die durch katalytische Hydrierung bzw. saure Hydrolyse mit Halogenwasserstoffsäuren, insbesondere aber mit Trifluoressigsäure wieder abgespalten werden.

Als Carboxylschutzgruppen sind beispielsweise Alkylester, und insbesondere Benzylester zu nennen, die sauer oder alkalisch abgespalten werden können. Die Benzylgruppen sind darüberhinaus besonders schonend durch Hydrogenolyse zu entfernen.

Als Aminosäuren werden die in großen Mengen durch Hydrolyse von Proteinen erhältlichen "natürlichen" L-Aminosäuren bevorzugt, jedoch können für bestimmte Zwecke auch synthetisch hergestellte racemische oder D-Aminosäuren oder in der Natur nicht vorkommende Aminosäuren verwendet werden. Weiterhin kann statt einer Aminosäure auch ein entsprechendes Peptid eingesetzt werden.

Die Erfindung wird durch folgende Beispiele näher erläutert.

### Beispiel 1:

Kopplung von 3-Amino-allo-saccharose mit Aminosäuren, die reine Kohlenwasserstoff-Seitenketten enthalten
3-(L-Leucyl-amino)-allo-saccharose
200 mg 3-Amino-allo-saccharose werden zusammen mit 137 mg N-BOC-L-Leucin (0,059 mmol) in 13 ml Lösungsmittel (Pyridin/ H₂O, 4:1) gelöst und im Eisbad gekühlt. Unter Kühlung wird anschließend DCC (1,0 mmol gelöst in 0,5 ml Lösungsmittel) unter Rühren und Kühlung langsam zugetropft. Nach einiger Zeit wird die Kühlung beendet. Nach ca. 17stündiger Reaktionszeit (mit Rührung) wird die Reaktion durch Zugabe eines Tropfen Eisessig zur Reaktionsmischung abgebrochen und nach 15minütigem Rühren der entstandene unlösliche Dicyclohexylharnstoff abfiltriert. Das Lösungsmittel wird unter Zusatz von Toluol schonend am Rotationsverdampfer (40 °C, Vakuum) entfernt. Das Reaktionsprodukt wird mit Wasser und Diethylether in einen Schütteltrichter überführt und die wäßrige Phase mehrmals mit Diethylether extrahiert. Das Reaktionsprodukt in der wäßrigen Phase wird gefriergetrocknet und das Produkt mittels Säulenchromatographie (stat. Phase: Kieselgel, Eluent: Essigsäureethylester/ Ethanol/Wasser, 5:3:1) gereinigt. Man erhält 3-(N-t-BOC-L-Leucyl-amino)-allo-saccharose in 35 %iger Ausbeute.
C₂₃H₄₂N₂O₁₃ (M= 554 g/mol)
¹³C-NMR-Daten (75,5 MHz, D₂O/Aceton-d₆):
=178,0 (C1"), 158,3 (C7"), 105,0 (C2'), 93,0 (C1), 82,6 (C5'), 82,2 (C8"), 77,5 (C3'), 74,6 (C4'), 69,4/66,1/65,7 (C2,4,5), 62,9/62,4 (C6',1'), 60,8 (C6), 55,3/53,4 (C3,2"), 40,8 (C3"), 28,5 (C9"-11"), 25,1 (C4"), 23,1 (C5", 6")
FAB-Massenspektrum (pos., Matrix Glycerin):
m/z= 555[M+H]⁺, 393[M-Fructosylrest+H]⁺, 761[M+2Glycerin+Na]⁺ Die Abspaltung der t-Butyloxycarbonyl(BOC)-Schutzgruppe erfolgt mit 90 %iger Trifluoresssigsäure: Hierzu wird das Reaktionsprodukt in kalter 90 %iger Trifluoressigsäure gelöst und 20 bis 30 min bei 4 °C stehen gelassen. Anschließend wird die Substanz durch Zugabe von kaltem, trockenen Diethylether ausgefällt, abfiltriert und mehrmals mit Di-ethylether nachgewaschen. Nach säulenchromatographischer Reinigung erhält man 3-(L-Leucyl-amino)-allo-saccharose.

In gleicher Weise werden hergestellt:
3-(N-BOC-L-Alanyl-amino)-allo-saccharose C₂₀H₃₆N₂O₁₃ (M= 512 g/mol)
¹³C-NMR-Daten (75,5 MHz, D₂O/Aceton-d₆):
δ=170,8 (C1"), 154,9 (C4"), 104,2 (C2'), 91,6 (C1), 82,7 (C5'), 78,2 (C5"), 76,4 (C3'), 73,7 (C4'), 69,0/65,5/65,0 (C2,4,5), 61,8 (C6',1'), 60,1 (C6), 55,3 (C3), 49,5 (C2"), 28,1 (C6"-8"), 17,9 (C3")

3-(N-BOC-L-Phenylalanyl-amino)-allo-saccharose C₂₆H₄₀N₂O₁₃ (M= 588 g/mol)
¹³C-NMR-Daten (75,5 MHz, D₂O/Aceton-d₆:
δ=176,3 (C1"), 158,0 (C10"), 137,0 (C4"), 128,9/128,5/ 128,1 (C5"-9"), 104,5 (C2'), 92,5 (C1), 82,4 (C5'), 81,0 (C11"), 77,4 (C3'), 74,3 (C4'), 69,2/65,7/65,4 (C2,4,5), 66,5 (C6'), 62,4 (C1'), 60,5 (C6), 56,0/53,0 (C3,2"), 40,5 (C3"), 28,1 (C12"-14")

FAB-Massenspektrum (pos., Matrix Glycerin):
m/z= 589[M+H]⁺, 427[M-Fructosylrest+H]⁺, 611[M+Na]⁺
Auch aus diesen Verbindungen kann die BOC-Gruppe mit Trifluoressigsäure abgespalten werden.

### Beispiel 2

Kopplung von 3-Amino-allo-saccharose mit sauren und basischen Aminosäuren
3-(4-L-Aspartyl-amino)-allo-saccharose C₁₆H₂₈N₂O₁₃ (M= 456 g/mol)
Dazu werden 150 mg 3-Amino-allo-saccharose (0,44 mmol) zusammen mit 142 mg N-t-BOC-L-Asparaginsäure- -benzylester in 13 ml Lösungsmittel (Pyridin/H₂O, 4:1) gelöst und im Eisbad gekühlt. Unter Kühlung wird anschließend DCC (0,7 mmol gelöst in 0,5 ml Lösungsmittel) unter Rühren und Kühlung langsam zugetropft. Nach einiger Zeit wird die Kühlung beendet. Nach ca. 17stündiger Reaktionszeit (mit Rührung) wird die Reaktion durch Zugabe eines Tropfens Eisessig zur Reaktionsmischung abgebrochen und nach 15minütigem Rühren der entstandene unlösliche Dicyclohexylharnstoff abfiltriert. Das Lösungsmittel wird unter Zusatz von Toluol schonend am Rotationsverdampfer (40 °C, Vakuum) entfernt. Das Reaktionsprodukt wird mit Wasser und Diethylether in einen Schütteltrichter überführt und die wäßrige Phase mehrmals mit Diethylether extrahiert. Das Reaktionsprodukt in der wäßrigen Phase wird gefriergetrocknet.

Die Abspaltung der Benzyl-Schutzgruppe an der α-Carboxylgruppe des Asparaginsäure-Rests erfolgt durch Hydrogenolyse. Hierzu wird das gefriergetrocknete Rohprodukt (200 mg) in einem Dreihalskolben mit Rückflußkühler in 22 ml Methanol gelöst. Die Lösung wird in Gegenwart eines Katalysators (Palladium/Aktivkohle, 10 % Pd) unter Rühren 3 h bei einer Temperatur von 30 bis 40 °C mit durchstömendem Wasserstoff behandelt, nachdem vorher der Luftsauerstoff durch Stickstoff verdrängt worden ist. Nach Abfiltrieren des Katalysators und Entfernen des Lösungsmittels am Rotationsverdampfer (30 °C, Vakuum) wird das Produkt mittels Säulenchromatographie (stat. Phase: Kieselgel, Eluent: Essigsäureethyl-ester/Ethanol/Wasser, 5:3:1) gereinigt. Man erhält 3-(N-BOC-4-L-Aspartylamino)-allo-saccharose in 44 %iger Ausbeute.

Die Abspaltung der BOC-Schutzgruppe erfolgt mit 90 %iger Trifluoresssigsäure: Hierzu wird das Reaktionsprodukt in 0,7 ml kalter 90 %iger Trifluoressigsäure gelöst und 15 bis 20 min bei 4 °C stehen gelassen. Anschließend wird die Substanz durch Zugabe von kaltem, trockenen Diethylether ausgefällt, abfiltriert und mehrmals mit Diethylether nachgewaschen. Nach säulenchromatographischer Reinigung erhält man 3-(4-L-Aspartyl-amino)-allo-saccharose (77 % Ausbeute).

¹³C-NMR-Daten (75,5 MHz, D₂O/Aceton-d₆):
δ=174,7/174,1 (C1", 4"), 104,8 (C2'), 93,2 (C1), 82,7 (C5'), 77,3 (C3'), 74,9 (C4'), 69,6/66,2/65,9 (C2,4,5), 63,2/62,3 (C6', 1'), 61,1 (C6), 53,5/52,5 (C3,2"), 37,3 (C3")

FAB-Massenspektrum (pos., Matrix Glycerin):
m/z= 457[M+H]⁺, 295[M-Fructosylrest+H]⁺, 913[2M+H]⁺

In gleicher Weise wird 3-(Na -BOC-N -CBZ-L-Lysyl)amino-allo-saccharose C₃₁H₄₉N₃O₁₅ (M=703 g/mol) hergestellt.

¹³C-NMR-Daten (75,5 MHz, D₂O/Aceton-d₆):
δ =176,3 (C1"), 158,0/157,9 (C7", 12"), 137,0 (C14"), 128,9/128,1/128,0 (C15"-19"), 104,5 (C2'), 92,5 (C1), 82,4 (C5'), 81,0 (C8"), 77,4 (C3'), 74,3 (C4'), 69,2/65,7/65,4 (C2,4,5), 66,5 (C6'), 62,4 (C1') 60,5 (C6), 56,1/53,0 (C3,2"), 40,5/40,6 (C3"-6", 13"), 28,1 (C9"-11")

FAB-Massenspektrum (pos., Matrix Glycerin):
m/z= 726[M+Na]⁺

Durch Abspaltung der Schutzgruppen mit Trifluoressigsäure und katalytische Hydrierung erhält man daraus 3-(L-Lysyl)amino-allo-saccharose.

### Beispiel 3

Hydrolytische Spaltung der Glycosidbindung der 3-(4-L-Aspartyl-amino)-allo-saccharose
Durch saure Hydrolyse erhält man aus der 3-(4-L-Aspartylamino)-allo-saccharose die 3-(4-L-Aspartyl-amino)-allose. Hierzu werden 150 mg 3-(4-L-Aspartyl-amino)-allo-saccharose mit 5 ml Salzsäure (0,5 N) 10 min auf 60 °C erhitzt. Anschließend wird die Reaktionslösung sofort auf 20 °C abgekühlt und mit Natronlauge neutralisiert. Die Abtrennung des Produkts von dem Reaktionsgemisch erfolgt mittels präparativer Flüssigchromatographie an einem Kationenaustauscher in der Ca²⁺-Form (Eluent: H₂O, 70 °C). 3-(4-L-Aspartyl-amino)allose wird mit einer Ausbeute von 80 % bezogen auf die eingesetzte 3-(4-L-Aspartyl-amino)-allo-saccharose erhalten.

## Patentansprüche

1. 3-(Aminoacyl-amino)-saccharide der allgemeinen Formel I, 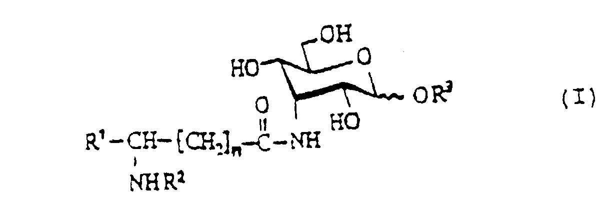
in der R¹ Wasserstoff, eine Carboxylgruppe oder eine Phenylgruppe oder eine Alkylgruppe mit 1 - 10 C-Atomen, die ggf. durch eine Phenyl-, Carboxyl-, Hydroxyl-, Mercapto- oder Aminogruppe substituiert ist,
wobei die genannten Substituenten ggf. mit Schutzgruppen versehen sind, bedeutet,
R² Wasserstoff oder eine in der Peptidchemie übliche Aminoschutzgruppe oder eine Peptidgruppe ist und
R³ Wasserstoff oder einen Fructosylrest darstellt,
n = 0 oder 1 ist.

2. Saccharid, ausgewählt aus den folgenden Verbindungen:
3-(4-L-Aspartyl-amino)-3-desoxy-D-allopyranosyl-β-D-fructofuranosid
3-(L-Alanyl-amino)-3-desoxy-D-allopyranosyl-β-D-fructofuranosid
3-(L-Leucyl-amino)-3-desoxy-D-allopyranosyl-β-D-fructofuranosid
3-(L-Lysyl-amino)-3-desoxy-D-allopyranosyl-β-D-fructofuranosid
3-(L-Phenylalanyl-amino)-3-desoxy-D-allopyranosyl-β-D-fructofuranosid
3-(4-L-Aspartyl-amino)-3-desoxy-D-allopyranose

3. Verfahren zur Herstellung der Substanzen gemäß Anspruch 1 oder 2, **dadurch gekennzeichnet**, daß man 3-Amino-allo-saccharose mit einer entsprechend geschützten Aminosäure der Formel II umsetzt 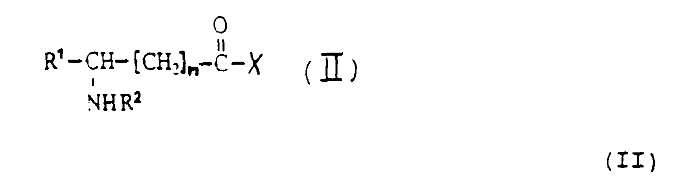 in welcher R¹, R² und n die obige Bedeutung haben, und X eine aktivierende Gruppe darstellt,
ggf. die Fructosylgruppe und/oder vorhandene Schutzgruppen abspaltet und das 3-(Aminoacyl-amino)-saccharid isoliert.

4. Verfahren nach Anspruch 3, **dadurch gekennzeichnet**, daß die Zwischen- und Endprodukte chromatographisch gereinigt werden.

## Claims

1. 3-(Aminoacyl-amino)-saccharides of the general formula I, 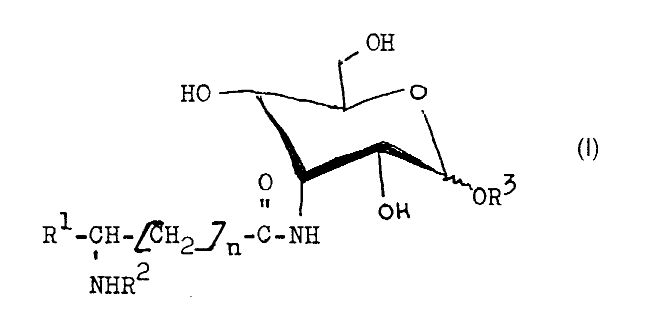
in which R¹ signifies hydrogen, a carboxyl group or a phenyl group or an alkyl group with 1 - 10 C-atoms which is optionally substituted by a phenyl, carboxyl, hydroxyl, mercapto or amino group, whereby the said substituents are optionally provided with protective groups,
R² is hydrogen or an amino protective group conventional in peptide chemistry or a peptide group, and
R³ represents hydrogen or a fructosyl residue,
n = 0 or 1.

2. Saccharide selected from the following compounds:
3-(4-L-aspartyl-amino)-3-desoxy-D-allopyranosyl-β-D-fructofuranoside
3-(L-alanyl-amino)-3-desoxy-D-allopyranosyl-β-D-fructofuranoside
3-(L-leucyl-amino)-3-desoxy-D-allopyranosyl-β-D-fructofuranoside
3-(L-lysyl-amino)-3-desoxy-D-allopyranosyl-β-D-fructofuranoside
3-(L-phenylalanyl-amino)-3-desoxy-D-allopyranosyl-β-D-fructofuranoside
3-(4-L-aspartyl-amino)-3-desoxy-D-allopyranose

3. Process for the preparation of substances according to claim 1 or 2, characterised in that one reacts 3-amino-allo-saccharose with a correspondingly protected amino acid of the formula II 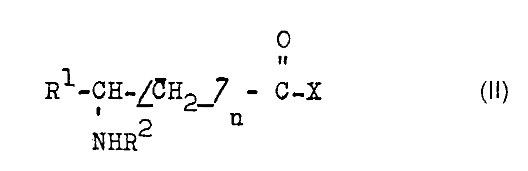 in which R¹, R² and n have the above meaning and X represents an activating group, optionally splits off the fructosyl group and/or protective groups present and isolates the 3-(aminoacyl-amino)-saccharide.

4. Process according to claim 3, characterised in that the intermediate and end products are purified chromatographically.

## Revendications

1. 3-(aminoacyl-amino)-saccharides répondant à la formule générale I 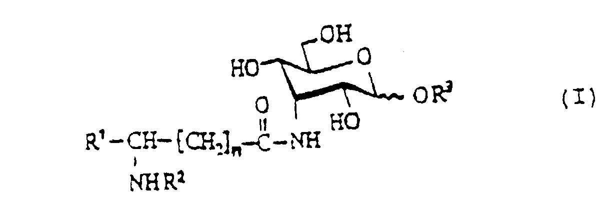
dans laquelle R¹ représente un atome d'hydrogène, un groupe carboxyle ou un groupe phényle ou encore un groupe alkyle contenant de 1 à 10 atomes de carbone, qui porte le cas échéant un ou plusieurs substituants phényle, carboxyle, hydroxyle, mercapto ou amino,
les substituants mentionnés portant le cas échéant des groupes de protection,
R² représente un atome d'hydrogène ou encore un groupe protecteur du groupe amino habituel dans la chimie des peptides ou représente un groupe peptide, et
R³ représente un atome d'hydrogène ou un résidu fructosyle,
n = 0 ou 1.

2. Saccharide choisi parmi les composés ci-après:
le 3-(4-L-aspartyl-amino)-3-désoxy-D-allopyranosyl-β-D-fructofuranoside,
le 3-( L-alanyl-amino)-3-désoxy-D-allopyranosyl-β-fructofuranoside,
le 3-(L-leucyl-amino)-3-désoxy-D-allopyranosyl-β-D-fructofuranoside,
le 3-(L-lysyl-amino)-3-désoxy-D-allopyranosyl-β-D-fructofuranoside,
le 3-( L-phénylalanyl-am ino)-3-désoxy-D-al lopyranosyl-β-D-fructofuranoside,
le 3-(4-L-aspartyl-amino)-3-désoxy-D-allopyranose.

3. Procédé pour la préparation des substances selon la revendication 1 ou 2, caractérisé en ce qu'on fait réagir du 3-amino-allosaccharose avec un acide aminé protégé de manière correspondante, répondant à la formule II 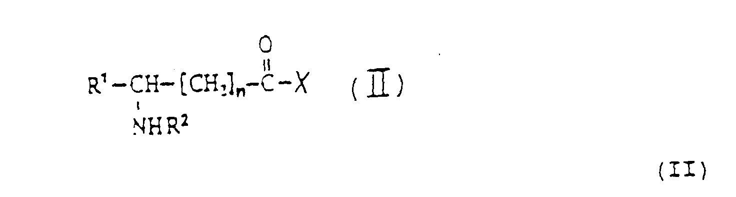 dans laquelle R¹, R² et n ont la signification indiquée ci-dessus et X représente un groupe d'activation,
le cas échéant, on élimine le groupe fructosyle et/ou les groupes protecteurs présents, et on isole le 3-(aminoacyl-amino)-saccharide.

4. Procédé selon la revendication 3, caractérisé en ce qu'on purifie par chromatographie les produits intermédiaires et les produits finals.
